# EUROPÄISCHE PATENTSCHRIFT

(11) **EP 3 822 244 B1**
(45) Veröffentlichungstag und Bekanntmachung des Hinweises auf die Patenterteilung: **02.12.2026**
(21) Anmeldenummer: 20202551.6
(22) Anmeldetag: 19.10.2020
(51) Int. Cl.: C07C 5/25, C07C 11/08, B01J 21/12, B01J 35/61

(54) **VERFAHREN ZUR ISOMERISIERUNG VON OLEFINEN**
METHOD FOR ISOMERISATION OF OLEFINS
PROCÉDÉ D'ISOMÉRISATION DES OLÉFINES

(30) Priorität: 14.11.2019 EP 19209078
(43) Veröffentlichungstag der Anmeldung: 19.05.2021
(73) Patentinhaber: Evonik Oxeno GmbH & Co. KG, 45772 Marl (DE)
(72) Erfinder: Geilen, Frank, 45721 Haltern am See (DE); Gärtner, Felix, 45721 Haltern am See (DE); Zanthoff, Horst-Werner, 45481 Mülheim a.d. Ruhr (DE); Stochniol, Guido, 45721 Haltern am See (DE)
(74) Vertreter: Evonik Patent Association

(56) Entgegenhaltungen:
- WO-A1-2012/123292
- DE-A1- 10 123 950
- US-A1- 2018 170 833
- US-A1- 2018 208 524

## Beschreibung

Die vorliegende Erfindung betrifft ein Verfahren zur Isomerisierung von C4- bis C9-Olefinen mit innenständiger Doppelbindung zu den entsprechenden Olefinen mit endständiger Doppelbindung unter Verwendung eines heterogenen Katalysatorsystems, welches eine Silicium-Aluminium-Mischoxidzusammensetzung umfasst.

Bei Isomerisierungen (bei Vorgängen innerhalb eines Moleküls spricht man auch von Umlagerungen) wird das Ausgangsmolekül in ein Molekül unveränderter Summenformel, aber geänderter Atomfolge, Atomanordnung oder Bindungsanordnungen überführt. Isomere besitzen oft vergleichbare Bindungsenergien, wodurch eine relativ freie Umwandlung ineinander stattfinden kann. Man unterscheidet, je nach Umwandlungsart z.B. die Bindungsisomerisierung, bei der Doppelbindungen beispielsweise zwischen C-C Bindungen umgelagert werden (wobei aber auch zahlreiche Bindungsisomerisierungen mit Beteiligung von Heteroatomen, wie O, N, P oder S dem Fachmann bekannt sind), die Skelettisomerisierung, bei der lineare Verbindungen in verzweigte umgelagert werden, die Hydroisomerisierung, bei der ein Alkan in Gegenwart von Wasserstoff über die Zwischenstufe eines Alkens in ein isomeres Alkan umgewandelt wird, oder die cis/trans Isomerisierung, bei der die Substituenten einer Doppelbindung umgelagert werden. Isomerisierungen werden häufig durch azide/basische Katalysatoren beschleunigt. Die Eigenschaften der Katalysatoren, wie die Stärke der Säure/Base-Zentren, entscheiden wesentlich, welche der Isomerisierungen in einem Molekül stattfinden. Die hier gewünschte Isomerisierung ist eine Bindungsisomerisierung.

Olefine mit endständiger Doppelbindung, sogenannte alpha-Olefine (z. B. 1-Buten, 1-Hexen oder 1-Octen), sind in der chemischen Industrie wichtige Ausgangsstoffe für zahlreiche Verfahren, wie beispielsweise der Hydroformylierung zur Herstellung von Aldehyden, der Oligomerisierung oder der Polymerisation. Olefine mit endständiger Doppelbindung haben im Vergleich zu Olefinen mit innenständiger Doppelbindung (z. B. 2-Buten, 2-Hexen oder 2-Octen) den Vorteil, dass sie eine teils deutlich höhere Reaktivität in den industriell betriebenen Verfahren aufweisen. Zudem sind die Olefine mit endständiger Doppelbindung deutlich teurer als die entsprechenden Olefine mit innenständiger Doppelbindung. Im Gegensatz dazu gibt es in der chemischen Industries aber auch einen Bedarf an Olefinen mit innenständiger Doppelbindung.

Die entsprechenden Olefine mit endständiger oder innenständiger Doppelbindung können über verschiedene Verfahren, beispielsweise Crackingverfahren, bereitgestellt werden. Eine andere Möglichkeit ist die katalytische Isomerisierung von Olefinen mit innenständiger Doppelbindung zu den entsprechenden Olefinen mit endständiger Doppelbindung. Möglich ist auch die umgekehrte Isomerisierung zu Olefinen mit innenständiger Doppelbindung. Der Grad der Umwandlung ist jeweils durch das thermodynamische Gleichgewicht limitiert. Die katalytische Isomerisierung zu Olefinen mit endständiger Doppelbindung ist beispielsweise aus der EP 0 718 036 A1 bekannt.

Die im Stand der Technik für die Isomerisierung zu Olefinen mit endständiger Doppelbindung eingesetzten Katalysatoren sind üblicherweise Katalysatoren mit Säure/Base-Funktionalität und Übergangsmetall-haltige Katalysatoren (letztere teils in Gegenwart von Wasserstoff, was einer sogenannten Hydroisomerisierung entspricht). Bei den Säure/Base-Katalysatoren finden Alkali- und Erdalkalimetall-dotierte Alumosilikate, entsprechend ausgetauschte Zeolithe oder reine basische Oxide (z.B. MgO) Anwendung.

US 2018/208524 A1 beschreibt ein Verfahren zur Herstellung von Propylen ausgehend von 2-Butene. Das 2-Buten wird dabei innerhalb des Verfahrens zu 1-Buten unter Verwendung eines MFI-strukturierten Alumosilikat-Katalysators isomerisiert und anschließend einer Metathese unterworfen wird.

Die DE 101 239 50 A1 offenbart Granulate auf Basis von mittels Aerosol mit Aluminiumoxid dotiertem, pyrogen hergestelltem Siliziumdioxid mit bestimmten physikalisch-chemischen Kenndaten.

Das allgemeine Problem von Isomerisierungsreaktionen ist, dass die zu isomerisierenden Olefine aufgrund ihrer Doppelbindung reaktive Moleküle sind und deshalb Nebenreaktionen auftreten können. Ein Beispiel ist die Oligomerisierung, die an sauren Katalysatorsystem stattfinden kann und bei Einsatz entsprechend von sauren Katalysatoren bei der Isomerisierung als Nebenreaktion auftritt. Um ein Oligomerisieren der Olefine bei der Isomerisierung zu Olefinen mit endständiger Doppelbindung zu verhindern, werden eher basische Katalysatorsystem oder mit Alkali- oder Erdalkalimetallen dotierte Katalysatoren eingesetzt.

Überraschenderweise wurden gefunden, dass, abweichend davon, auch (schwach) saure, SiO₂-basierte Katalysatoren mit einem gewissen Aluminiumoxidanteil, also beispielsweise Katalysatoren basierend auf den hier genannten Silicium-Aluminium-Mischoxidzusammensetzungen, eingesetzt werden können, die bei einer guten Produktselektivität eine hohe Aktivität für die Isomerisierung liefern. Zudem tritt keine bzw. nahezu keine Oligomerisierung als Nebenreaktion auf.

Das erfindungsgemäße Verfahren ist demzufolge ein Verfahren zur Isomerisierung von C4- bis C9-Eduktolefinen mit innenständiger Doppelbindung, vorzugsweise C4- bis C8-Eduktolefinen mit innenständiger Doppelbindung, weiterhin bevorzugt C4- bis C6-Eduktolefinen mit innenständiger Doppelbindung, besonders bevorzugt C4-Eduktolefinen mit innenständiger Doppelbindung zu Produktolefinen mit endständiger Doppelbindung unter Verwendung eines heterogenen Katalysators, wobei der Katalysator eine Silicium-Aluminium-Mischoxidzusammensetzung umfasst.

Die als Katalysator eingesetzte Silicium-Aluminium-Mischoxidzusammensetzung kann mittels Flammenhydrolyse nach dem unter anderem in der DE 198 47 161 A1 oder der EP 0 850 876 A1 offenbarten Verfahren hergestellt werden. Bei diesem sogenannten "co-fumed-Verfahren" werden flüchtige Silizium- und Aluminium-Verbindungen, z. B. Siliciumtetrachlorid und Aluminiumtrichlorid, in eine Knallgasflamme aus Wasserstoff und Sauerstoff bzw. Luft eingesprüht, wodurch die Silizium- und Aluminium-Verbindungen mittels des in der Knallgasflamme entstandenen Wassers hydrolysiert werden und sich die Mischoxidzusammensetzung bildet.

Ein alternatives Verfahren, welches ebenfalls in den genannten Schriften offenbart ist, ist das sogenannte Dotierverfahren. Dabei wird in der Knallgasflamme einerseits ein Oxid, hier beispielsweise Siliziumoxid, aus seiner flüchtigen Verbindung (z.B. Siliciumtetrachlorid) durch Flammenhydrolyse erzeugt und in die Knallgasflamme zusätzlich ein Aerosol eingespeist, in dem sich ein Salz des zu dotierenden Elementes, hier beispielsweise Aluminium, befindet und so das entsprechende Mischoxid bildet. Die so flammenhydrolytisch hergestellten Silicium-Aluminium-Mischoxidzusammensetzung ist überwiegend bis vollständig amorph.

Die mittels der exemplarisch genannten Herstellverfahren hergestellten Silicium-Aluminium-Mischoxidzusammensetzung zeichnen sich durch ihre hohe chemische Reinheit aus und weisen vorzugsweise die nachfolgende Zusammensetzung auf:
a) 98,5 bis 99,95 Gew.-% Siliciumoxid (gerechnet als SiO₂); und
b) 0,05 bis 1,5 Gew.-% Aluminiumoxid (gerechnet als Al₂O₃).

In einer bevorzugten Ausführungsform der vorliegenden Erfindung enthält die Silicium-Aluminium-Mischoxidzusammensetzung zusätzlich Alkali- und/oder Erdalkalimetalloxide, besonders bevorzugt in einer Menge von bis zu 1 Gew.-% bezogen auf die Gesamtzusammensetzung. Um die Alkali- oder Erdalkalimetalloxide einzubringen, kann die flammenhydrolytisch hergestellte Mischoxidzusammensetzung mit einer wässrigen Alkalimetall- oder Erdalkalimetallhydroxid-Lösung behandelt werden. Das kann beispielsweise durch Tränken oder Imprägnieren des flammenhydrolytisch hergestellten Mischoxidzusammensetzung mit einer Alkali- und/oder Erdalkalimetallsalz-Lösung erfolgen. Anschließend wird die der behandelte Mischoxidzusammensetzung mit Wasser gewaschen, bei 100 bis 150°C getrocknet und bei 300 bis 600°C, bevorzugt bei 450 bis 550°C, kalziniert. Silicium- und Aluminiumoxide können an sich auch bereits Spuren von Alkali- oder Erdalkalimetallen enthalten, die hier unberücksichtigt bleiben.

Die erfindungsgemäß eingesetzten Silicium-Aluminium-Mischoxidzusammensetzungen können zusätzlich mit einer sauren, wässrigen Lösung mit einer Phosphorquelle behandelt werden. Als Phosphorquelle kann Phosphorsäure, Phosphonsäure, Phosphinsäure, Polyphosphorsäure oder Dihydrogenphosphat, bevorzugt Phosphorsäure, eingesetzt werden. Dazu wird die Mischoxidzusammensetzung zunächst in Wasser suspendiert wird und die entstandene Suspension anschließend mit der Phosphorquelle versetzt, vorzugsweise so, dass der pH-Wert im Bereich von 0 bis 6, weiterhin bevorzugt im Bereich von 1 bis 2.5, besonders bevorzugt im Bereich von 2 bis 2,5 liegt. Anschließend wird die der behandelte Mischoxidzusammensetzung mit Wasser gewaschen, bei 100 bis 150°C getrocknet und bei 300 bis 600°C, bevorzugt bei 450 bis 550°C, kalziniert.

In einer bevorzugten Ausführungsform liegt die erfindungsgemäß eingesetzte Silicium-Aluminium-Mischoxidzusammensetzung überwiegend (d. h. > 70 %) oder vollständig in Form von aggregierten Primärpartikeln vor. Die Silicium-Aluminium-Mischoxidzusammensetzung zeichnet sich dabei unter anderem dadurch aus, dass das Gewichtsverhältnis (Al₂O₃/SiO₂)_{Oberfläche} der Primärpartikel im oberflächennahen Bereich kleiner ist als das Gewichtsverhältnis (Al₂O₃/SiO₂)_{gesamt} im gesamten Primärpartikel. Der Begriff "oberflächennaher Bereich" meint dabei den Bereich von der Oberfläche bis zu einer Tiefe von 5 nm. Der Unterschied in den Gewichtsverhältnissen bedeutet, dass die Aluminiumoxidkonzentration an der Oberfläche kleiner ist als in der gesamten Zusammensetzung. Der gesamte Primärpartikel schließt den Anteil an Siliciumdioxid und Aluminiumoxid im oberflächennahen Bereich mit ein.

Bevorzugt ist demnach eine Silicium-Aluminium-Mischoxidzusammensetzung, die überwiegend oder vollständig in Form von aggregierten Primärpartikeln vorliegt, bei denen
I) das Gewichtsverhältnis (Al₂O₃/SiO₂)_{gesamt} im Gesamtprimärpartikel 0,003 bis 0,015, bevorzugt 0,005 bis 0,01 beträgt; und
II) das Gewichtsverhältnis (Al₂O₃/SiO₂)_{Oberfläche} der Primärpartikel im oberflächennahen Bereich kleiner ist als im Gesamtprimärpartikel.

Das Gewichtsverhältnis (Al₂O₃/SiO₂)_{Oberfläche} auf der Oberfläche kann zum Beispiel durch röntgeninduzierte Photoelektronen-Spektroskopie (XPS-Analyse) des Pulvers bestimmt werden. Zusätzliche Information über die Oberflächenzusammensetzung kann durch energiedispersive Röntgenstrahlung (TEM-EDX-Analyse) von einzelnen Primärpartikeln bestimmt werden. Das Gewichtsverhältnis (Al₂O₃/SiO₂)_{gesamt} im Gesamtprimärpartikel kann durch chemische oder physikalisch-chemische Methoden, z.B. Röntgenfluoreszenzanalyse, des Pulvers bestimmt werden

Die als Katalysator verwendete Silicium-Aluminium-Mischoxidzusammensetzung ist röntgen-amorph. Röntgen-amorph im Sinne der vorliegenden Erfindung meint, dass eine röntgen-amorphe Substanz im Röntgendiffraktogramm bis zur Nachweisgrenze von 5 nm keine kristalline Struktur aufweist.

Die erfindungsgemäß beschriebene Silicium-Aluminium-Mischoxidzusammensetzung insbesondere mit der oben genannten Zusammensetzung und insbesondere mit den genannten Unterschieden in den Gewichtsverhältnissen (Al₂O₃/SiO₂) weist vorzugsweise eine BET-Oberfläche 100 bis 220 m²/g auf (bestimmt gemäß DIN ISO 9277 ((Stand: 2014-01)).

Weiterhin kann es vorteilhaft sein, wenn die Silicium-Aluminium-Mischoxidzusammensetzung eine Dibutylphthalat-Zahl, in g Dibutylphthalat (DBP)/100 g Mischzusammensetzung, von 300 bis 350 aufweist. Die DBP-Zahl stellt ein Maß für die Struktur von Aggregaten dar. Niedrige Zahlen korrespondieren mit einer niedrigen Struktur, hohe Zahlen mit einer hohen Struktur. Der beschriebene Bereich von 300 bis 350 für die erfindungsgemäße Mischoxidzusammensetzung entspricht einer hohen Struktur. Bei der DBP-Absorption wird die Kraftaufnahme, beziehungsweise das Drehmoment (in Nm), der rotierenden Schaufeln des DBP-Messgerätes bei Zugabe definierter Mengen von DBP gemessen. Dabei ergibt sich für die Silicium-Aluminium-Mischoxidzusammensetzung vorzugsweise ein scharf ausgeprägtes Maximum mit einem anschließenden Abfall bei einer bestimmten Zugabe von DBP. Die Dibutylphthalatabsorption kann beispielsweise mit einem Gerät RHEOCORD 90 der Fa. Haake, Karlsruhe gemessen werden. Hierzu werden 12 g des Silicium-Aluminium-Mischoxidpulvers in eine Knetkammer eingefüllt, diese mit einem Deckel verschlossen und Dibutylphthalat über ein Loch im Deckel mit einer vorgegebenen Dosierrate von 0,0667 ml/s eindosiert. Der Kneter wird mit einer Motordrehzahl von 125 Umdrehungen pro Minute betrieben. Nach Erreichen des Drehmomentmaximums wird der Kneter und die DBP-Dosierung automatisch abgeschaltet. Aus der verbrauchten Menge DBP und der eingewogenen Menge der Partikel wird die DBP-Absorption berechnet nach: DBP-Zahl (g/100 g) = (Verbrauch DBP in g / Einwaage Pulver in g) x 100.

Für die industriell betriebene Isomerisierung unter Verwendung eines Katalysators, der die Silicium-Aluminium-Mischoxidzusammensetzung umfasst, ist eine Reaktionsführung in einem oder mehreren Festbettreaktoren bevorzugt. Für Flüssigphasenreaktionen können auch Slurry-Reaktoren oder Trickle-Bed-Reaktoren eingesetzt werden. Es können auch andere Reaktortypen, wie Wirbelbettreaktoren oder Wanderbettreaktoren, eingesetzt werden. Dazu ist es erforderlich, dass die zuvor beschriebene, flammhydrolytisch bzw. pyrogen hergestellte Mischoxidzusammensetzung unter Zugabe eines Bindemittels mittels eines dem Fachmann bekannten formgebenden Verfahrens in Form gebracht wird, insbesondere in Form von Granulaten, Pellets oder Formkörpern, wie Tabletten, Zylindern, Kugeln, Strangextrudaten oder Ringen. Geeignete Bindemittel sind dem Fachmann bekannt, beispielswiese Tonerde, keramische Tonen, Kolloiden oder auch amorphe Zeolithe Verwendung finden.

Zur Formgebung werden zunächst 1 bis 20 Gew.-% der Silicium-Aluminium-Mischoxidzusammensetzung mit einem der vorgenannten Bindemittel und zusätzlich mit temporären Hilfsstoffen, wie beispielsweise Wasser, wässrigen Lösungen, Wasserersatzstoffen, wie Glykolen oder Polyglykolen, und optional weiteren Hilfsstoffen, wie Fixierungsmitteln, beispielsweise Celluloseethern, und/oder Plastifizierungsmitteln, beispielsweise Polysacchariden, und/oder Presshilfsmitteln, beispielsweise nichtionischen Wachsdispersionen, vermischt. Dieser Vorgang kann in dem Fachmann bekannten Vorrichtungen, beispielsweise in einem Kneter oder einem Intensivmischer, durchgeführt werden. Anschließend findet die eigentliche Formgebung durch ein formgebendes Verfahren, wie beispielsweise Pelletierung, Extrusion oder Trockenpressen, statt. Vor dem Einbau in den oder die Festbettreaktoren werden die Formen bzw. Formkörper in einem Temperaturbereich von 200 bis 700°C kalziniert, wodurch zumindest die temporären Hilfsstoffe entfernt werden.

Die Silicium-Aluminium-Mischoxidzusammensetzung kann auf einem für die Isomerisierung inerten Träger, beispielsweise einem Metall-, Kunststoff- oder Keramikträger, aufgebracht werden. Ist die Silicium-Aluminium-Mischoxidzusammensetzung auf einem inerten Träger aufgebracht, wird die Masse und Zusammensetzung des inerten Trägers bei der Bestimmung der Zusammensetzung der Silicium-Aluminium-Mischoxidzusammensetzung nicht berücksichtigt.

Das erfindungsgemäße Verfahren wird mit der vorgenannt beschriebenen Silicium-Aluminium-Mischoxidzusammensetzung als Katalysator durchgeführt, um C4- bis C9-Eduktolefinen mit innenständiger Doppelbindung, vorzugsweise C4- bis C8-Eduktolefinen mit innenständiger Doppelbindung, weiterhin bevorzugt C4- bis C6-Eduktolefinen mit innenständiger Doppelbindung, besonders bevorzugt C4-Eduktolefinen mit innenständiger Doppelbindung, zu Produktolefinen mit endständiger Doppelbindung zu isomerisieren.

Die Olefine werden nicht unbedingt in reiner Form eingesetzt, sondern in technisch verfügbaren Kohlenwasserstoffmischungen. Durch die Isomerisierung wird der Gehalt des Produktolefins demzufolge in der Kohlenwasserstoffmischung erhöht und gleichzeitig der Gehalt an Eduktolefin verringert.

C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem C₄-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Kohlenwasserstoffmischung zugeführt. Geeignete C4-olefinhaltige Stoffströme sind beispielsweise das Rohbutan oder das C4-Raffinat III. Rohbutan fällt unter anderem bei der Oligomerisierung von C4-Olefinen als Nebenprodukt an. Ein Rohbutan im Sinne der vorliegenden Erfindung ist aber jeder C4-Kohlenwasserstoffstrom, der neben einem hohen Anteil gesättigter C4-Kohlenwasserstoffe (typischerweise mehr als 50 Gewichts-%) noch lineare Butene enthält, wobei der 1-Butengehalt unter 10% der linearen Butene ausmacht. C4-Raffinat III im Sinne der Erfindung fällt nach der Abtrennung von zumindest 1,3-Butadien, Isobuten und 1-Buten aus C4-Fraktionen von Steam- oder FCC-Crackern an.

Die Eduktolefine sind erfindungsgemäß Olefine mit innenständiger Doppelbindung, die durch die Isomerisierung zumindest teilweise in Produktolefine, also Olefine mit endständiger Doppelbindung (alpha-Olefin) ist, umgewandelt werden. In einer bevorzugten Ausführungsform handelt es sich bei den Eduktolefinen um cis- und/oder trans-2-Butene bzw. Kohlenwasserstoffmischungen, die cis- und/oder trans-2-Butene enthalten, die durch die erfindungsgemäße Isomerisierung in 1-Buten umgewandelt werden. Das ermöglicht die Anreicherung von 1-Buten in eher 1-Buten-armen Kohlenwasserstoffmischungen, um diese wertschöpfend nutzbar zu machen, beispielsweise für die Weiterverarbeitung in Prozessen die hohe 1-Butenkonzentrationen bevorzugen (z. B. die Oligomerisierung oder die oxidative Dehydrierung zu Butadien.), für die 1-Buten-Gewinnung oder für die Hydroformylierung zu Pentanal.

Der Umsatz der Eduktolefine bzw. des Eduktolefins zum Produktolefin ist insbesondere durch die temperaturabhängige Lage des chemischen Gleichgewichts der Isomerisierungsreaktion nach oben begrenzt. Der Vorteil durch die Verwendung eines erfindungsgemäßen Katalysators ist, dass der Umsatz dem thermodynamischen Gleichgewichtsumsatz in einem breiteren Temperaturbereich entspricht oder diesen nur geringfügig unterschreitet. Dies gilt auch im Hinblick auf die Isomerisierung von 2-Buten zu 1-Buten, die durch das thermodynamische Gleichgewicht der n-Buten-Isomere begrenzt ist. Das thermodynamische Gleichgewicht eines 2-Butene und 1-Buten enthaltenen Gemisches wird durch hohe Temperaturen in Richtung des 1-Butens verschoben. Das thermodynamische Gleichgewicht für 1-Buten liegt bei einer Temperatur von 400 °C bei etwa zwischen 24 % und 25 % und bei einer Temperatur von 500 °C bei etwa 29 %.

Für das erfindungsgemäße Isomerisierungsverfahren wird vorzugsweise mindestens ein Festbettreaktor eingesetzt. Es können auch andere Reaktortypen, wie Wirbelbettreaktoren, Wanderbettreaktoren, Slurry-Reaktoren oder Trickle-Bed-Reaktoren eingesetzt werden.

Das erfindungsgemäße Verfahren kann bei Atmosphärendruck durchgeführt werden. Es können jedoch auch höhere Reaktionsdrücke angewandt werden. Die Druckfahrweise im erfindungsgemäßen Verfahren ist beispielsweise sinnvoll, wenn das Produktolefin aus dem erfindungsgemäßen Isomerisierungsverfahren noch einer Trennstufe zugeführt wird, die ebenfalls unter Druck betrieben wird. So kann man die Kohlenwasserstoffmischung nach erfolgter Isomerisierung von 2-Buten zu 1-Buten noch einer Trennstufe zuführen, in der 1-Buten und 2-Buten voneinander unter Druck destillativ getrennt werden.

Die erfindungsgemäße Isomerisierung von 2-Buten zu 1-Buten erfolgt vorzugsweise bei einer Temperatur zwischen 20 °C und 600 °C, weiterhin bevorzugt zwischen 100 °C und 500 °C und besonders bevorzugt zwischen 200 °C und 450 °C. Die Gas-Raumgeschwindigkeiten (gas hourly space velocity = GHSV) können von 2 000 bis 8 000 h⁻¹, vorzugsweise von 2 500 bis 4 000 h⁻¹. Die Selektivität der erfindungsgemäßen Isomerisierung bezüglich des Produktolefins ist vorzugsweise größer als 80%, weiterhin bevorzugt größer als 82% und besonders bevorzugt größer als 85% ist.

Bei Nachlassen der Aktivität und Selektivität des erfindungsgemäßen Katalysators infolge von Kohlenstoffablagerungen auf dem Katalysator wird dieser zweckmäßigerweise regeneriert. Ein vorteilhaftes Verfahren zur Katalysatorregenerierung besteht darin, dass man die Kohlenstoffablagerungen auf dem desaktivierten Katalysator in Sauerstoff enthaltenden Gasen, vorzugsweise in Luft, abbrennt. Es kann zweckmäßig sein, hierbei die Luft mit Stickstoff zu verdünnen. Die Katalysatorregenerierung wird im Allgemeinen bei Temperaturen von 350 bis 600 °C, vorzugsweise von 400 bis 450 °C, durchgeführt. Hierdurch lassen sich in der Regel die Anfangsaktivität und die Anfangsselektivität des erfindungsgemäßen Katalysators in einfacher Weise zurückgewinnen.

Die Erfindung wird nachfolgend anhand eines Beispiels beschrieben. Dies dient der Erläuterung und stellt keine Einschränkung des Erfindungsgegenstands dar.

### Beispiel 1 - Isomerisierung von 2-Buten zu 1-Buten

0,2 g eines erfindungsgemäßen Katalysators (Aerosil^{®} MOX170, ca. 1 Gew.-% Aluminiumoxid, BET-Oberfläche zwischen 140 und 200 m²/g) wurden in einen Rohrreaktor mit einem Durchmesser von 0,6 mm eingefüllt. Der Reaktor wurde mit Rohbutan mit der nachfolgenden Zusammensetzung beschickt:

| 1-Buten | Cis-2-Buten | Trans-2-Buten | Iso-Buten | n-Butan | iso-Butan | Rest-KW |
|---|---|---|---|---|---|---|
| 1,56 | 8,87 | 20,18 | 0 | 67,9 | 0,53 | 0,86 |

Dabei wurde das Rohbutan mit unterschiedlichen Volumenströmen durch den Reaktor geleitet. Die Isomerisierung erfolgte bei einer Temperatur von 380 °C und bei einem Druck im Reaktor zwischen 5,7 bis 6 bara (bar absolut). Bei der Reaktion wurden die Umsätze von 2-Buten und die Selektivitäten für die Bildung von 1-Buten bestimmt. Die Analyse erfolgte durch GasChromatographie. Ausgewertet wurden die Peak-Flächen nach der Methode des internen Standards. Als interner Standard wurde n-Butan verwendet.

**Tabelle 1: Umsätze und Selektivitäten von Beispiel 1**

| Vol.-Strom Rohbutan | Umsatz 2-Buten | Selektivität Bildung 1-Buten |
|---|---|---|
| [g/h] | [%] | [%] |
| 7,5 | 24,91 | 87,82 |
| 15,2 | 21,91 | 90,66 |
| 25,2 | 20,51 | 94,44 |

Die Ergebnisse zeigen, dass die erfindungsgemäßen Katalysatoren sehr gut für die Isomerisierung geeignet sind.

## Patentansprüche

1. Verfahren zur Isomerisierung von C4- bis C9-Eduktolefinen mit innenständiger Doppelbindung zu Produktolefinen mit endständiger Doppelbindung bei einer Temperatur zwischen 200 °C und 450 °C unter Verwendung eines heterogenen Katalysators, wobei der Katalysator eine Silicium-Aluminium-Mischoxidzusammensetzung ist, die die nachfolgende Zusammensetzung aufweist:
a) 98,5bis 99,95 Gew.-% Siliciumoxid (gerechnet als SiO₂); und
b) 0,05 bis 1,5 Gew.-% Aluminiumoxid (gerechnet als Al₂O₃),
wobei der Katalysator röntgen-amorph ist und
wobei die Silicium-Aluminium-Mischoxidzusammensetzung eine BET-Oberfläche von 100 bis 220 m²/g aufweist.

2. Verfahren nach Anspruch 1, wobei der Katalysator aus Formkörpern besteht, die aus der Silicium-Aluminium-Mischoxidzusammensetzung unter Zugabe von Bindemitteln und zumindest temporären Hilfsstoffen in einem formgebenden Verfahren hergestellt wurden.

3. Verfahren nach Anspruch 1 oder 2, wobei bei der Isomerisierung eine Kohlenwasserstoffmischung eingesetzt wird, die das zu isomerisierende Eduktolefin und auch bereits das Produktolefin enthält, wobei der Gehalt des Produktolefins durch die Isomerisierung erhöht wird.

4. Verfahren nach einem der Ansprüche 1 bis 3, wobei bei Gas-Raumgeschwindigkeit bei der Isomerisierung von 2 000 bis 8 000 h⁻¹, vorzugsweise von 2 500 bis 4 000 h⁻¹ beträgt.

5. Verfahren einem der Ansprüche 1 bis 4, wobei als Eduktolefine C4- bis C8-Eduktolefinen mit innenständiger Doppelbindung, vorzugsweise C4- bis C6-Eduktolefinen mit innenständiger Doppelbindung, besonders bevorzugt C4-Eduktolefinen mit innenständiger Doppelbindung eingesetzt werden.

6. Verfahren nach Anspruch 5, wobei als Eduktolefine cis- und/oder trans-2-Buten bzw. Kohlenwasserstoffmischungen, die diese 2-Butene enthalten, eingesetzt werden und das Produktolefin mit endständiger Doppelbindung 1-Buten ist.

7. Verfahren nach einem der Ansprüche 1 bis 6, wobei die Silicium-Aluminium-Mischoxidzusammensetzung überwiegend oder vollständig in Form von aggregierten Primärpartikeln vorliegt.

8. Verfahren nach Anspruch 7, wobei die Silicium-Aluminium-Mischoxidzusammensetzung sich dadurch auszeichnet, dass das Gewichtsverhältnis (Al₂O₃/SiO₂)_{Oberfläche} der Primärpartikel im oberflächennahen Bereich kleiner ist als das Gewichtsverhältnis (Al₂O₃/SiO₂)_{gesamt} im gesamten Primärpartikel.

9. Verfahren nach Anspruch 7 oder 8, wobei die Silicium-Aluminium-Mischoxidzusammensetzung überwiegend oder vollständig in Form von aggregierten Primärpartikeln vorliegt, bei denen
I) das Gewichtsverhältnis von (Al₂O₃/SiO₂)_{gesamt} im Gesamtprimärpartikel 0,003 bis 0,015, bevorzugt 0,005 bis 0,01 beträgt; und
II) das Gewichtsverhältnis (Al₂O₃/SiO₂)_{Oberfläche} der Primärpartikel in einer oberflächennahen Schicht mit einer Dicke von 5 nm kleiner als im Gesamtprimärpartikel ist.

## Claims

1. Process for the isomerization of C4 to C9 reactant olefins having an internal double bond to product olefins having a terminal double bond at a temperature of between 200°C and 450°C using a heterogeneous catalyst, the catalyst being a silicon-aluminium mixed oxide composition having the following composition:
a) 98.5% to 99.95% by weight of silicon oxide (calculated as SiO₂); and
b) 0.05% to 1.5% by weight of aluminium oxide (calculated as Al₂O₃),
wherein the catalyst is X-ray amorphous and
wherein the silicon-aluminium mixed oxide composition has a BET surface area of 100 to 220 m²/g.

2. Process according to Claim 1, wherein the catalyst consists of shaped bodies produced from the silicon-aluminium mixed oxide composition in a forming process with the addition of binders and at least temporary auxiliaries.

3. Process according to Claim 1 or 2, wherein the isomerization is carried out using a hydrocarbon mixture that comprises the reactant olefin to be isomerized and additionally already the product olefin, wherein the content of the product olefin is increased through isomerization.

4. Process according to any of Claims 1 to 3, wherein the gas hourly space velocity during the isomerization is from 2000 to 8000 h⁻¹, preferably from 2500 to 4000 h⁻¹.

5. Process according to any of Claims 1 to 4, wherein the reactant olefins used are C4 to C8 reactant olefins having an internal double bond, preferably C4 to C6 reactant olefins having an internal double bond, more preferably C4 reactant olefins having an internal double bond.

6. Process according to Claim 5, wherein the reactant olefins used are cis- and/or trans-2-butene or hydrocarbon mixtures comprising these 2-butenes, and the product olefin having a terminal double bond is 1-butene.

7. Process according to any of Claims 1 to 6, wherein the silicon-aluminium mixed oxide composition is predominantly or entirely present in the form of aggregated primary particles.

8. Process according to Claim 7, wherein the silicon-aluminium mixed oxide composition is **characterized in that** the weight ratio (Al₂O₃/SiO₂)_{surface} of the primary particles in the near-surface region is less than the weight ratio (Al₂O₃/SiO₂)ₜₒₜₐₗ in the totality of the primary particles.

9. Process according to Claim 7 or 8, wherein the silicon-aluminium mixed oxide composition is predominantly or entirely present in the form of aggregated primary particles in which
I) the weight ratio (Al₂O₃/SiO₂)ₜₒₜₐₗ in the totality of the primary particles is 0.003 to 0.015, preferably 0.005 to 0.01; and
II) the weight ratio (Al₂O₃/SiO₂)_{surface} of the primary particles in a near-surface layer having a thickness of 5 nm is less than in the totality of the primary particles.

## Revendications

1. Procédé pour l'isomérisation d'oléfines de départ en C4 à C9 comportant une double liaison interne en des oléfines produites comportant une double liaison terminale à une température comprise entre 200 °C et 450 °C en utilisant un catalyseur hétérogène, le catalyseur étant une composition d'oxyde mixte de silicium-aluminium ayant la composition suivante :
a) 98,5 à 99,95 % en poids d'oxyde de silicium (calculé sous forme de SiO₂) ; et
b) 0,05 à 1,5 % en poids d'oxyde d'aluminium (calculé sous forme d'Al₂O₃),
dans lequel le catalyseur est amorphe aux rayons X et
dans lequel la composition d'oxyde mixte de silicium-aluminium présente une surface BET de 100 à 220 m²/g.

2. Procédé selon la revendication 1, le catalyseur étant constitué de corps moulés qui ont été fabriqués à partir de la composition d'oxyde mixte de silicium-aluminium par un procédé de mise en forme avec ajout de liants et d'auxiliaires au moins temporaires.

3. Procédé selon la revendication 1 ou 2, dans lequel un mélange d'hydrocarbures est utilisé lors de l'isomérisation qui contient l'oléfine de départ à isomériser ainsi que déjà l'oléfine produite, la teneur en oléfine produite étant augmentée par l'isomérisation.

4. Procédé selon l'une quelconque des revendications 1 à 3, la vitesse spatiale de gaz lors de l'isomérisation étant de 2 000 à 8 000 h⁻¹, de préférence de 2 500 à 4 000 h⁻¹.

5. Procédé selon l'une quelconque des revendications 1 à 4, des oléfines de départ en C4 à C8 présentant une double liaison interne, de préférence des oléfines de départ en C4 à C6 présentant une double liaison interne, et de manière particulièrement préférée des oléfines de départ en C4 présentant une double liaison interne, étant utilisées en tant qu'oléfines de départ.

6. Procédé selon la revendication 5, du cis- et/ou trans-2-butène ou des mélanges d'hydrocarbures qui contiennent ces 2-butènes étant utilisés comme oléfines de départ et l'oléfine produite présentant une double liaison terminale étant du 1-butène.

7. Procédé selon l'une quelconque des revendications 1 à 6, la composition d'oxyde mixte de silicium-aluminium étant présente majoritairement ou entièrement sous forme de particules primaires agrégées.

8. Procédé selon la revendication 7, la composition d'oxyde mixte de silicium-aluminium **se caractérisant en ce que** le rapport pondéral (Al₂O₃/SiO₂)_{surface} des particules primaires dans la région proche de la surface est inférieur au rapport pondéral (Al₂O₃/SiO₂)ₜₒₜₐₗ dans l'ensemble de la particule primaire.

9. Procédé selon la revendication 7 ou 8, la composition d'oxyde mixte de silicium-aluminium étant présente majoritairement ou entièrement sous forme de particules primaires agrégées, dans lesquelles
I) le rapport pondéral de (Al₂O₃/SiO₂)ₜₒₜₐₗ dans l'ensemble de la particule primaire est de 0,003 à 0,015, de préférence de 0,005 à 0,01 ; et
II) le rapport pondéral (Al₂O₃/SiO₂)_{surfacee} des particules primaires dans une couche proche de la surface présentant une épaisseur de 5 nm est inférieur à celui dans l'ensemble de la particule primaire.
